# EUROPEAN PATENT APPLICATION

(11) **EP 2 177 628 A2**
(43) Date of publication of application: **21.04.2010**
(21) Application number: 09179309.1
(22) Date of filing: 12.09.2006
(51) Int. Cl.: C12Q 1/68, C12N 15/28, G01N 33/574, A61B 5/00

(54) **Markers for diagnosis of cancer and its use**

(30) Priority: 12.09.2005 KR 20050084864
(62) Divisional of application: 06798748.7
(71) Applicant: Daewoong Co., Ltd., Gyunggi-do 462-120 (KR)
(72) Inventor: Shin, InKyung, 446-954, Gyeonggi-do (KR); Park, Hyun jin, 442-090, Gyeonggi-do (KR); Choi, Joo young, 449-814, Gyeonggi-do (KR); Hyun, Hyae Jung, 449-814, Gyeonggi-do (KR); Shin, Kyeong-Sun, 449-814, Gyeonggi-do (KR); Park, Seung kook, 463-030, Gyeonggi-do (KR)
(74) Representative: MacLean, Martin Robert

(57) **Abstract**

Disclosed herein are diagnostic markers CTHRC1, CANP and KIAA0101, which are overexpressed specifically in breast or colorectal cancer. A method for diagnosing the cancer by detecting the markers, and a method for preventing or treating by inhibiting the expression and activity of the markers are also disclosed.

SEQUENCE LISTING

<110>    DAEWOONG CO., LTD.

<120>    Markers for Diagnosis of Cancer and Its Use

<130>    P32068EP-PCT

<140>    EP06798748.7
<141>    2006-09-12

<150>    KR 10-2005-0084864
<151>    2005-09-12

<160>    22

<170>    KopatentIn 1.71

<210>    1
<211>    16
<212>    DNA
<213>    Artificial Sequence

<220>
<223>    forward primer

<400>    1
ccgcgggaat tcgatt                                                    16

<210>    2
<211>    18
<212>    DNA
<213>    Artificial Sequence

<220>
<223>    reverse primer

<400>    2
gccgcgaatt cactagtg                                                  18

<210>    3
<211>    18
<212>    DNA
<213>    Artificial Sequence

<220>
<223>    ACTB-specific froward primer

<400>    3
ggcattgccg acaggatg                                                  18

<210>    4
<211>    24
<212>    DNA
<213>    Artificial Sequence

<220>
<223>    ACTB-specific reverse primer

<400>    4
ctcaggagga gcaatgatct tgat                                           24

<210>    5
<211>    20
<212>    DNA

<213>    Artificial Sequence

<220>
<223>    CTHRC1-specific forward primer


<400>    5
ttgggaaaat tgcggagtgt                                                    20


<210>    6
<211>    20
<212>    DNA
<213>    Artificial Sequence

<220>
<223>    CTHRC1-specific reverse primer


<400>    6
gccgaagtga gccactgaac                                                    20


<210>    7
<211>    24
<212>    DNA
<213>    Artificial Sequence

<220>
<223>    CANP-specific forward primer


<400>    7
tcggtctgac ataggtgaat ttga                                               24


<210>    8
<211>    22
<212>    DNA
<213>    Artificial Sequence

<220>
<223>    CANP-specific reverse primer


<400>    8
tcatccacca tggactgttt tc                                                 22


<210>    9
<211>    22
<212>    DNA
<213>    Artificial Sequence

<220>
<223>    KIAA0101-specific forward primer


<400>    9
tcatcgagga aagctgaaaa ta                                                 22


<210>    10
<211>    20
<212>    DNA
<213>    Artificial Sequence

<220>
<223>    KIAA0101-specific reverse primer


<400>    10
aattcctttt tgccacttgg                                                    20

<210>    11
<211>    25
<212>    DNA
<213>    Artificial Sequence

<220>
<223>    forward primer for cloning of CTHRC1


<400>    11
ctgaccacgt tcctctcctc ggtct                                              25


<210>    12
<211>    28
<212>    DNA
<213>    Artificial Sequence

<220>
<223>    reverse primer for cloning of CTHRC1


<400>    12
tgtcatttaa gtgaaccatt ccaaggca                                           28


<210>    13
<211>    24
<212>    DNA
<213>    Artificial Sequence

<220>
<223>    forward primer for cloning of CANP1


<400>    13
ccttgtttct ccatcttatc gagt                                               24


<210>    14
<211>    24
<212>    DNA
<213>    Artificial Sequence

<220>
<223>    reverse primer for cloning of CANP1


<400>    14
attttctccc attccgtagg ttgt                                               24


<210>    15
<211>    26
<212>    DNA
<213>    Artificial Sequence

<220>
<223>    forward primer for cloning of KIAA0101


<400>    15
ctcggctggg aagtcagttc gttctc                                             26


<210>    16
<211>    27
<212>    DNA
<213>    Artificial Sequence

<220>

<223>    reverse primer for cloning of KIAA0101

<400>    16
aagccactgt gcccaccatg attctat                                          27

<210>    17
<211>    25
<212>    RNA
<213>    Artificial Sequence

<220>
<223>    CTHRC1-specific siRNA

<400>    17
uuauagcuuc aaugggaaga ggucc                                            25

<210>    18
<211>    25
<212>    RNA
<213>    Artificial Sequence

<220>
<223>    CTHRC1-specific siRNA

<400>    18
ggaccucuuc ccauugaagc uauaa                                            25

<210>    19
<211>    25
<212>    RNA
<213>    Artificial Sequence

<220>
<223>    CANP-specific siRNA

<400>    19
ccagagucug auacagucua agaaa                                            25

<210>    20
<211>    25
<212>    RNA
<213>    Artificial Sequence

<220>
<223>    CANP-specific siRNA

<400>    20
uuucuuagac uguaucagac ucugg                                            25

<210>    21
<211>    25
<212>    RNA
<213>    Artificial Sequence

<220>
<223>    KIAA0101-specific siRNA

<400>    21
augaaacuga ugucgaauua guggc                                            25

<210>    22

<211>    25
<212>    RNA
<213>    Artificial Sequence

<220>
<223>    KIAA0101-specific siRNA


<400>    22
gccacuaauu cgacaucagu uucau                                              25

## Description

### Technical Field

The present invention relates to markers for the diagnosis of cancer. More particularly, the present invention relates to diagnostic markers CTHRC1, CANP and KIAA0101 for cancer, which are overexpressed particularly in the event of breast cancer and colorectal cancer. Also, the present invention relates to a method for diagnosing cancer by detecting the expression of the diagnostic markers and a method for the prophylaxis and treatment of cancer, comprising the inhibition of the expression and activity of the diagnostic markers.

### Background Art

Three million or more persons in the world were reported to have newly contracted cancer in 2003. In Korea, the incidence of cancer increases every year, amounting to hundreds of thousands of people in 2002. Lung cancer, colorectal cancer and breast cancer make up about 40% of all cancer cases. The incidence of colorectal cancer, breast cancer and lung cancer is tending to gradually increase in Korea as the diet is becoming westernized.

In spite of extensive and intensive research into cancer for years, no therapeutics capable of certainly curing cancer have been developed yet. The operative elimination of cancer in an early stage is known to be the most effective method of increasing the chances of long-term survival.

Depending on the mechanism, conventional anticancer drugs can be largely divided into adjunct therapies, cytotoxics, and hormones. Adjunct therapies do not directly target tumor cells, but are used to assist primary treatment, and are exemplified by Anti-emetics, bisphosphonates, hematopoietic growth factors, analgesics, and the like. Cytotoxic therapies act on rapidly proliferating cells to induce them into apoptosis. They can be divided into alykylating agents, anti-metabolites, vika alkaloids, and anthracyclines/etoposides, depending on the active mechanism thereof. Since the use thereof as the first anticancer drug, cytotoxin therapies have been widely used in the treatment of various cancers for the last 50 years. However, non-specific active mechanisms allow the cytotoxic therapies to have inhibitory activity not only against tumor cells, but also exhibit strong toxicity on other highly proliferating cells, thereby incurring significant side effects. Hormonal agents have been developed to kill the tumors of hormone-sensitive tissues, such as the breast, the prostate gland, etc. The cell division of tumor cells in such tissue as the breast or the prostate gland tends to be promoted by certain hormones. Accordingly, the suppression of the mechanism of these hormones could result in the inhibition of the progression of cancer. Hormonal therapy, although low in toxicity, also produces side effects. Upon the long-term administration of hormonal agents, in addition, there occur incurable cancers resistant to hormonal agents.

As alternatives to overcome the problems of conventional anticancer drugs, that is, side effects and resistance generation, novel therapies which act in a novel mechanism for specifically targeting cancer have been developed. Innovative therapies, although versatile in therapeutic mechanism, have something in common with one another in that they acts as tumor specific therapies by taking advantage of cancer specific markers or genetic abnormalities, which appear during the oncogenesis of normal cells. The therapeutics which are currently developed can be largely divided into angiogenesis inhibitors, immunostimulators, and tumor-targeted agents according to the cancer therapy mechanism. The most important point of the cancer targeted therapy, which is now arising as an alternative to minimize the toxicity of anticancer drugs, is to find genes specific for cancer cells. To accomplish this, the world's most eminent biotechnology companies have searched desperately for new target genes through gene expression profiling, gene mapping, proteomics, in silico analysis, etc. For example, Genentech, one of the leading biotechnology companies in the world, has advanced a project for novel target discovery using genomics for the last 27 years and possesses patent rights for genes associated with about 1,200 diseases. In recent years, the SPDI (Secreted Protein Discovery Initiative) project was conducted (1996-2001) to secure 1,000 or more secretary proteins including novel 250 proteins.

With this background, leading to the present invention, intensive and thorough research into cancer diagnosis and therapy, conducted by the present inventor, aiming to screen for differences in expression level between breast, colorectal, and lung cancer cells and normal cells, resulted in the finding that three novel genes CTHRC1, CANP and KIAA0101 are overexpressed specifically in breast or colorectal cancer cells. It was also observed that when the expression of these genes is suppressed, the cancer cells are induced to undergo apoptosis and hindered from proliferating. Therefore, the genes CTHRC1, CANP and KIAA0101 can be used not only as diagnostic markers of breast or colorectal cancer, but also as targets for cancer therapy.

### Disclosure

### Technical Problem

It is therefore an object of the present invention to provide a diagnostic marker for breast or colorectal cancer, comprising a gene selected from a group consisting of CTHRC1 (Collagen Triple Helix Repeat Containing 1), CANP (Cancer-Associated Nucleoprotein), KIAA0101 and combinations thereof.

It is another object of the present invention to provide a diagnostic maker for breast or colorectal cancer, comprising a protein selected from a group consisting of CTHRC1, CANP, KIAA0101 and combinations thereof.

It is another object of the present invention to provide a kit for detecting a diagnostic marker for breast or colorectal cancer, comprising an agent capable of measuring the mRNA level of a gene selected from a group consisting of CTHRC1, CANP, KIAA0101 and combinations thereof.

It is another object of the present invention to provide a kit for detecting a diagnostic marker for breast or colorectal cancer, comprising an agent capable of measuring the protein level of a gene selected from a group consisting of CTHRC1, CANP, KIAA0101 and combinations thereof.

It is another object of the present invention to provide a method for detecting a diagnostic marker for breast or colorectal cancer, comprising comparing expression levels of a gene selected from a group consisting of CTHRC1, CANP, KIAA0101 and combinations thereof between a test bio-specimen and a control specimen.

It is another object of the present invention to provide a pharmaceutical composition for the prophylaxis and treatment of breast or colorectal cancer, comprising an siRNA (small interfering RNA) sequence, capable of inducing RNA interference specific for a predetermined gene, composed of an antisense RNA strand complimentary to the mRNA of the predetermined gene and a corresponding sense RNA strand, the predetermined gene being selected from a group consisting of CTHRC1, CANP, KIAA0101 and combinations thereof.

It is another object of the present invention to provide a pharmaceutical composition for the prophylaxis or treatment of breast or colorectal cancer, comprising an antibody specific for a protein selected from a group consisting of CTHRC1, CANP, KIAA0101 and combinations thereof.

It is another object of the present invention to provide a method for suppressing the cell proliferation of breast or colorectal cancer or inducing apoptosis in breast or colorectal cancer cells, comprising reducing the intracellular mRNA or protein level or protein activity of a gene selected from a group consisting of CTHRC1, CANP, KIAA0101 and combinations thereof.

### Drawing Description

FIG. 1 shows DNA separated on DDRT PCR gel.
FIG. 2 shows DNA chip hybridization images
FIG. 3 shows real time PCR results in graph.
FIG. 4 shows an oncogenesis process in an animal.
FIG. 5 shows the specific gene suppression of RNAi through change in mRNA level.
FIG. 6 shows the specific gene suppression of RNAi through the induction of apoptosis and change in cell proliferation.
FIG. 7 shows the specific gene suppression of RNAi through DNA fragmentation.
FIG. 8 shows the specific gene suppression of RNAi through change in caspase activity.
FIG. 9 shows the specific gene suppression of RNAi through membrane alteration.
FIG. 10 shows the specific gene suppression of RNAi through change in cell cycle.

### Best Mode

In accordance with an embodiment, the present invention pertains to a marker for the diagnosis of breast or colorectal cancer, comprising a gene selected from a group consisting of CTHRC1 (Collagen Triple Helix Repeat Containing 1), CANP (Cancer-Associated Nucleoprotein), KIAA0101 and combinations thereof, or a protein corresponding to the gene.

The term "diagnosis" as used herein means the process of identifying a medical condition or disease by its signs and symptoms and from the results of various diagnostic procedures. For the purpose of the present invention, "diagnosis" is used to mean determining the incidence of breast or colorectal cancer by examining whether the diagnostic marker of breast or colorectal cancer is expressed.

The terms "marker for diagnosis", "diagnostic marker" or "diagnosis marker", as used herein, mean material capable of distinguishing breast or colorectal cancer cells from normal cells. As long as their levels are increased or decreased in breast or colorectal cancer cells compared to normal cells, organic bio molecules, such as polypeptides, nucleic acids (e.g., mRNA), lipids, glycolipids, glycoproteins, and saccharides (monosaccharides, disaccharides, oligosaccharides, etc) can be used as diagnostic markers of breast or colorectal cancer. The marker for the diagnosis of breast or colorectal cancer, useful in the present invention, is a gene selected from among CTHRC1, CANP, KIAA0101 and combinations thereof, or a corresponding protein, the expression level of which increases in breast or colorectal cancer cells as compared in normal cells.

CTHRC1 (Collagen Triple Helix Repeat Containing 1; GeneID 115908 NM_138455), which was cloned in 2003 after the completion of the Human Genome Project (Clark et al, 2003; Zhang and Henzel, 2004), is reported to increase in expression level upon artery injury and inhibit the accumulation of collagen matrix to promote cell migration (Pyagay et al, 2005). However, its precise functions remain unclear.

CANP (Cancer-Associated Nucleoprotein; GeneID 374393 NM_198947) is registered as a gene cloned from hepatoma cells by Liu et al. Its full length clone was cloned by Strausberg et al. (Strausberg et al., 2002). There has been no information on CANP with regard to its function and relation with cancer.

As for KIAA0101 (KIAA0101; GeneID 9768 NM_014736), its ORF (open reading frame) alone is reported (Nagase *et al*,. 1995). The function of KIAA0101 has yet to be determined. Millennium Pharmaceuticals, Inc. possesses a patent right for use thereof as a marker for prostate cancer (U.S. 6,355,430). Mizutani et al., reported that KIAA0101 is overexpressed in malignant prostate cancer (Mizutani et al, 2005). However, neither has the overexpression of KIAA0101 in breast or colorectal cancer been reported, nor has the carcinogenicity of KIAA0101 been observed to date.

The present inventor found that the mRNA level of CTHRC1 increases 10.9 fold in breast cancer cells and 22 fold in colorectal cancer compared to normal cells, that the mRNA level of CANP increases 11 fold in breast cancer cells and 2 fold in colorectal cancer cells compared to normal cells, and that the mRNA level of KIAA0101 increases 12.8 fold in breast cancer cells and 2.2 fold in colorectal cancer cells compared to normal cells.

Therefore, the quantification of the expression levels of CTHRC1, CANP and KIAA0101 could lead to determine the generation of breast or colorectal cancer.

In accordance with another embodiment, the present invention pertains to a kit for detecting a diagnostic marker of breast or colorectal cancer on the basis of the mRNA level of a gene selected from among CTHRC1, CANP, KIAA0101 and combinations thereof.

In detail, the kit for detecting a diagnostic marker of breast or colorectal cancer in accordance with the present invention comprises an agent for measuring the mRNA level of the gene.

The agent for measuring the mRNA level of the gene is preferably a pair of primers or a probe. Because the nucleotide sequences are readily found in NM_138455(NCBI) for CTHRC1, NM_198947(NCBI) for CANP, and NM_014736(NCBI) for KIAA0101, those who are skilled in the art can design primers or probes useful for specifically amplifying predetermined regions of the genes, on the basis of the nucleotide sequences.

As used herein, the term "primer" refers to a short, nucleic acid strand having a free 3' hydroxyl group, which forms a base pair with a complementary template so as to serve as a starting point for the production of a new template strand. DNA synthesis or replication requires a suitable buffer, proper temperatures, polymerizing enzyme (DNA polymerase, or reverse transcriptase), and four kinds of nucleotide triphosphates, in addition to primers. Specific for the marker gene CTHRC1, CANP or KIAA0101, the primers useful in the present invention are sense and antisense nucleic acids ranging in length from 7 to 50 nucleotides. The primers may be provided with additional characteristics which do not alter the basic role of the primers as starting points.

If necessary, the primer sequences of the present invention may have a label which can be detected directly or indirectly using spectroscopic, photochemical, biochemical, immunochemical or chemical means. Examples of the label include enzymes (e.g., horseradish peroxidase, alkaline phosphatase), radioisotopes (e.g., ³²P), fluorescent molecules, and chemical groups (e.g., biotin).

In the present invention, the term "a pair of primers" refers to one selected from all combinations of primer pairs, each consisting of a sense nucleotide sequence and an antisense nucleotide sequence. Preferable is a pair of primers which can provide specificity and sensitivity for analysis results. When the nucleotide sequences of the primers are not inconsistent with non-targeted sequences which exist in a sample and can be used to amplify only a targeted gene sequence without non-specific amplification, the primers can provide high specificity. The term "non-specific amplification" as used herein means the amplification of nucleotide sequences other than targeted nucleotide sequences, resulting from the hybridization of the primer with non-targeted sequences and subsequent extension of the hybridized primer.

As used herein, the term "probe" refers to a nucleic acid (e.g., DNA or RNA) fragment capable of specifically binding to mRNA, ranging in length from ones to hundreds of bases. The probe useful in the present invention is labeled so as to detect the presence or absence of a specific mRNA. The probe may be in the form of oligonucleotides, single stranded DNA, double stranded DNA, or RNA.

The primer or probe useful in the present invention can be chemically synthesized using a phosphoramidite solid support or other well-known technique. Its nucleotide sequence may be modified using various means known to the art. Illustrative, non-limiting examples of the modification include methylation, "capping", substitution of natural nucleotides with one or more homologues, and alternation between nucleotides, such as uncharged linkers (e.g., methyl phosphonate, phosphotriester, phosphoroamidate, carbamate, etc.) or charged linkers (e.g., phosphorothioate, phosphorodithioate, etc.).

Thanks to the disclosure of sequences of the targeted gene, artisans in the art can readily determine a combination of pairs of highly specific and sensitive primers in consideration of hybridization degrees among sequences within the targeted gene with the aid of a preexisting database. In a concrete embodiment of the present invention, the expression of CTHRC1 is detected in an mRNA level with primers of SEQ ID NOS.: 5 and 6, the expression of CANP with primers of SEQ ID NOS.: 7 and 8, and the expression of KIAA0101 with primers of SEQ ID NOS.: 9 and 10.

The detection kit according to the present invention comprises a composition, solution or apparatus composed of one or more components.

Preferably, the kit for detecting a diagnostic marker of breast or colorectal cancer is comprised of elements necessary for RT-PCR. This RT-PCR kit may comprise, in detail, a test tube or another suitable container, a reaction buffer (variable in pH and Mg concentration), deoxynucleotides (dNTPs), enzymes such as Taq-polymerase, reverse transcriptase and DNase, an RNAse inhibitor, DEPC-water, and deionized water, as well as respective pairs of primers specific for the marker genes. Optionally, a pair of primers specific for a control gene for quantification may be included in the kit.

Additionally, the kit for detecting a diagnostic marker of breast or colorectal cancer may preferably comprise elements necessary for DNA chip function. In a preferable embodiment of the present invention, a DNA chip kit may comprise a substrate, a gene or corresponding cDNA attached as a probe to the substrate, and optionally a quantification control gene or corresponding cDNA attached to the substrate.

In accordance with a further embodiment, the present invention is directed to a kit for diagnosing breast or colorectal cancer, comprising a material capable of quantifying the expression level of a protein selected from a group consisting of CTHRC1, CANP, KIAA0101 and combinations thereof.

In detail, the kit for diagnosing breast or colorectal cancer according to the present invention comprises an agent capable of determining protein levels.

A preferable agent for determining protein levels may be an antibody. Since the marker proteins CTHRC1, CANP and KIAA0101 are identified, the production of antibodies against them may be readily accomplished using well-known techniques. As long as it can specifically bind to the protein CTHRC1, CANP or KIAA0101, any antibody, irrespective of whether it is polyclonal, monoclonal, or recombinant, can be used in the present invention.

Polyclonal antibodies can be produced using a well-known method in which the protein CTHRC1, CANP or KIAA0101 is injected into a host mammal and polyclonal antibodies are obtained from a serum taken from the host mammal. Examples of the host mammal useful in the production of polyclonal antibodies include goats, rabbits, sheep, monkeys, horses, pigs, cows, dogs, etc.

As for the production of monoclonal antibodies, it may be achieved using a well-known hybridoma method (Kohler and Milstein (1976), referred to in European Jounral of Immunology 6:511-519, or a phage antibody library technique (Clackson et al, Nature, 352:624-628, 1991; Marks et al, J. Mol. Biol., 222:58, 1-597, 1991).

In addition, the antibodies useful in the present invention may be in a complete antibody form, consisting of two full-length light chains and two full-length heavy chains, or may be functional fragments of antibody molecules. The term "functional fragments of antibody molecules" means segments having at least an antigen-binding function, exemplified by Fab, F(ab'), F(ab') 2 and Fv.

Preferably, the kit for detecting the diagnostic marker in accordance with the present invention comprises elements necessary for performing ELISA. This ELISA kit comprises a reagent for detecting a bound antibody, which includes, for example, a labeled secondary antibody, chromophores, and an enzyme (e.g., conjugated with antibody) and the substrate thereof. Optionally, the kit may further comprise an antibody specific for a control protein for quantification.

In accordance with still another embodiment, the present invention is directed to a method for detecting a diagnostic marker of breast or colorectal cancer, featuring the comparison of expression levels of a gene selected from a group consisting of CTHRC1, CANP, KIAA0101 and combinations thereof between a test bio-specimen and a control specimen.

In the method, the expression level of the gene may be determined at the mRNA or protein level. In this regard, the separation of mRNA or protein from the bio-specimen can be achieved using a well-known process.

As used herein, the term "bio-specimen" refers to biological mass, in which the expression levels of the marker genes CTHRC1, CANP and/or KIAA0101 are changed upon the incidence of breast or colorectal cancer, examples of which include tissues, cells, whole blood, sera, saliva, phlegm, cerebrospinal fluid, and urine, but are not limited thereto.

Quantitative analysis methods of mRNA levels may be exemplified by RT-PCR, competitive RT-PCR, real-time RT-PCR, RNase protection assay, Northern blotting, and DNA chip methods, but are not limited thereto.

For the quantification of protein levels, Western blotting, ELISA, radioimmunoassay, radioimmunodiffusion, Auchteroni? Immunodiffusion, rocket immunoelectrophoresis, immunohistochemical staining, immunoprecipitation assay, complement fixation assay, FACS, and/or protein chip may be used, and these methods are not limitative, but illustrative.

Through the detection method, the expression level of the marker gene in a test sample is compared with that of the marker gene in a normal control to diagnose breast or colorectal cancer.

In accordance with still a further embodiment, the present invention is concerned with siRNA, which specifically acts on CTHRC1, CANP or KIAA0101.

In a preferable embodiment, the present invention provides an siRNA (small interfering RNA) sequence, capable of inducing CTHRCl-specific RNA interference, comprising an antisense RNA strand complimentary to the mRNA of CTHRC1 and a corresponding sense RNA strand. In another preferable embodiment, the present invention provides an siRNA sequence, capable of inducing KIAA0101-specific RNA interference, comprising an antisense RNA strand complimentary to the mRNA of KIAA0101 and a corresponding sense RNA strand.

As used herein, the term "siRNA" refers to double stranded RNA involved in the RNA interference pathway (RNAi) where the siRNA. interferes with the expression of a targeted gene through cleavage of the mRNA thereof. Therefore, siRNA is composed of a sense RNA strand having the same sequence as the mRNA of a targeted gene and an antisense RNA strand complimentary thereto. With the capacity to suppress the expression of a targeted gene, siRNA is usually used in a gene knockdown technique or gene therapy.

siRNA is not limited to the form of complete base pairs in the double RNA strands, but may be in partially unpaired forms resulting from mismatch (corresponding bases are not complementary) or bulginess (lack of corresponding bases in one strand). siRNA ranges in length from 10 to 80 bases, preferably from 15 to 60 bases, and more preferably from 20 to 40 bases. The terminal structure of siRNA may be blunt or cohesive. In the latter case, both a 3' overhang end and a 5' overhang end are available, with no limitation given to the number of cohesive bases. For example, the cohesive end may be composed of 1 to 8 bases, preferably 2 to 6 bases. Additionally, siRNA may comprise low-molecular RNA (e.g., natural RNA molecules such as tRNA, rRNA, and viral RNA, or artificial RNA molecules) at the overhang part on one end, as long as it can maintain the expression interference effect on the targeted gene. As for the terminal structure of the siRNA, it may not be a truncated form at both ends, but may be a stem loop structure in which the terminal sites at one end of the double stranded RNA are connected to each other via a linker RNA. As long as it is sufficient to enable the formation of pairs in the stem, the length of the linker is not particularly limited.

siRNA can be introduced into cells through the transfection of in-vitro synthesized siRNA into cells or through the transfection or infection of an siRNA expression vector or a PCR-derived siRNA expression cassette, which is designed to express siRNA within cells.

When used in association with siRNA in the present invention, the term "specific" or "specifically" involves the capacity of inhibiting only a target gene, but not other genes, within cells. In the present invention, siRNA is CTHRC1, CANP or KIAA0101 specific.

As long as it can specifically reduce the mRNA level of CTHRC1, CANP or KIAA0101, the siRNA of the present invention is not limited, particularly in sequence or length. In a preferable embodiment of the present invention, nucleotide sequences of SEQ ID NOS.: 17 and 18 are provided for siRNA for CTHRC1, nucleotide sequences of SEQ ID NOS.: 19 and 20 for siRNA for CANP, and nucleotide sequences of SEQ ID NOS.: 21 and 22 for siRNA for KIAA0101. Each siRNA is observed to reduce the cellular expression level of the corresponding gene, induce apoptosis, and inhibit cell proliferation. Hence, the siRNA according to the present invention is useful in the prophylaxis or treatment of breast or colorectal cancer.

In accordance with yet another embodiment, the present invention is concerned with a pharmaceutical composition for the prophylaxis or treatment of breast or colorectal cancer, comprising the siRNA which specifically acts on the mRNA of a gene selected from among CTHRC1, CANP, KIAA0101 and combinations thereof.

Optionally, the composition based on the siRNA specific for the gene may further comprise an apoptosis suppressant. In addition, an agent for promoting the introduction of siRNA into a cell may be further included in the composition. This agent may be a promoter for the introduction of nucleic acids. For example, liposomes may be used alone or in combination with a lipophilic carrier selected from among sterols including cholesterol, cholate and deoxycholic acid. For the intracellular introduction of siRNA, cationic polymers, such as poly-L-lysine, spermine, polysilazane, PEI (polyethylenimine), polydihydroimidazolenium, polyallylamine, chitosan, etc. or anionic polymers, such as succinylated PLL, succinylated PEI, polyglutamic acid, polyaspartic acid, polyacrylic acid, polymethacrylic acid, dextran sulfate, heparin, hyaluronic acid, etc., may be used.

In still yet another embodiment, the present invention pertains to a pharmaceutical composition for the prophylaxis or treatment of breast or colorectal cancer, comprising an antibody specific for a protein selected from among CTHRC1, CANP, KIAA0101 and combinations.

When used in therapy, the antibody can be coupled (e.g., covalently bonded) with a preexisting drug directly or indirectly, that is, via a liker.

Examples of the drug coupled with antibodies include radionuclides, pharmaceuticals, lymphokine, toxins, and heterofunctional antibodies, but are not limited thereto. (1) Radionucleotides, such as ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ¹⁵³Sm, ¹²³I, and ¹¹¹In, (2) biological reaction modifiers or biological reaction-modifying drugs, such as methotrexate, adriamycin, and lymphokines including interferons, (3) toxins, such as ricin, abrin, and diphtheria, (4) heterofunctional antibodies, that is, complexes formed by conjugating heterotype antibodies with each other, which are able to bind both to cancer cells and to effector cells (e.g., killer cells such as T cells), and (5) natural, that is, non-related or non-complexed antibodies may be coupled with the antibody according to the present invention.

In accordance with a still yet further embodiment, the present invention pertains to a method for inhibiting the proliferation of breast or colorectal cancer cells or inducing the apoptosis of breast or colorectal cancer cells, featuring the reduction of the intracellular mRNA and protein level of a gene selected from among CTHRC1, CANP, KIAA0101 and combinations thereof or the activity of a corresponding protein.

Through this method, breast or colorectal cancer can be prevented and treated.

In an experiment conducted by the present inventor, the injection of a cell line overexpressing the gene CTHRC1, KIAA0101, or CANP into nude mice was found to form tumors, which implies that the genes CTHRC1, KIAA0101 and CANP play a direct and important role in oncogenesis as well as in the survival and proliferation of cancer cells. In addition, it was also observed that the reduction of the intracellular mRNA or protein level of the gene or the suppression of protein activity resulted in the induction of apoptosis of the cancer cells and a reduction in the proliferation of the cells.

Materials capable of reducing the intracellular mRNA or protein level of the gene or suppressing the activity of the protein may be single organic or inorganic compounds; polymeric compounds, such as proteins (e.g., antibodies), carbohydrates, nucleic acid molecules (e.g., antisense nucleotides, siRNA), and lipids; ribozyme; and complexes consisting of a plurality of compounds, as long as they specifically act on CTHRC1, CANP or KIAA0101.

In a preferable modification of this embodiment, the method for the prophylaxis or treatment of breast or colorectal cancer features the administration of a composition comprising an siRNA or antibody which acts specifically on CTHRC1, CANP or KIAA0101, so as to suppress cell proliferation or induce apoptosis in the cancer cells.

The composition comprising the siRNA or antibody may be formulated, along with a pharmaceutically acceptable carrier, into various dosage forms, such as tablets, troches, capsules, elixirs, suspensions, syrups, wafers, injections, etc.

The material capable of reducing the intracellular mRNA or protein level of the gene or suppressing the activity of the protein may be administered in a therapeutically or prophylactically effective amount. The dose of the active material may vary with various factors, including the kind and severity of disease, the age, sex, body weight, and drug sensitivity of patients, the kind of therapy, administration route, targeted cell, etc., and can be readily determined by one skilled in the art. The material capable of reducing the intracellular mRNA or protein level of the gene or suppressing the activity of the protein may be used alone or in combination with a conventional drug. In the latter case, the material and the drug may be administered simultaneously or sequentially. The administration may be single or multiple. It is important to administer the material in a dose suitable to obtain maximum therapeutic or prophylactic effect without side effects. In consideration of all of the above-mentioned factors, one skilled the art can readily determine the minimum dose sufficient to obtain the optimal effect.

As used herein, the term "administration" means the introduction of a predetermined material into patients using a suitable method. As long as it guarantees the transmission of the administered material to a targeted tissue, any administration route may be taken. For example, the composition of the present invention may be administered intraperitoneally, intravenously, intramuscularly, subcutaneously, orally, locally, intranasally, intrapulmonarily, intrarectally, or through other routes. Also, the pharmaceutical composition of the present invention may be administered with the aid of a device for guiding the active material to a targeted cell.

A better understanding of the present invention may be obtained in light of the following examples which are set forth to illustrate, but are not to be construed to limit the present invention.

### Mode for Invention

### EXAMPLES

### EXAMPLE 1: Cloning of Partial Genes Related to Cancer Using Differential Display (DD) RT-PCR

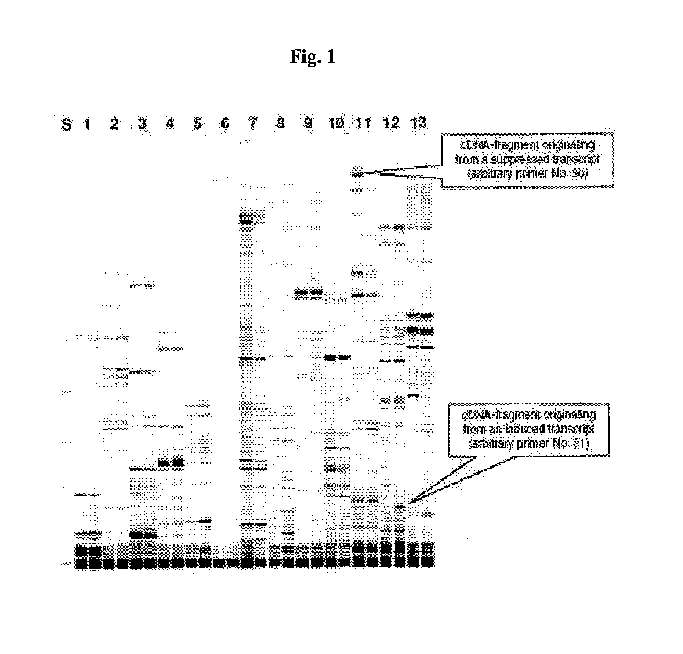

In order to clone novel genes related with breast, colorectal or lung cancer, the expression patterns of genes in cancer tissues were compared with those of genes in normal tissue using a differential display technique. Genes that exhibited a difference in expression pattern were selectively isolated (FIG. 1). As a result, 1,495 clones were detected to be related with breast cancer, 1,183 clones with colorectal cancer, and 597 clones with lung cancer.

### Step 1) Tissue sampling

Colorectal cancer tissue samples were taken from 9 Korean adult male patients and 6 Korean adult female patients, who were in various progression stages (St. Mary's Hospital, Korea). Breast cancer tissue samples were taken from 14 Korean adult female patients with breast cancer (St. Mary's Hospital, Korea). Lung cancer tissue samples were taken from 7 Korean male patients with NSCLC (non-small cell lung cancer) (St. Mary's Hospital, Korea). A set of biopsy samples was recovered from tumorous tissue and normal tissue around the tumorous tissue in each patient.

### Step 2) Total RNA isolation

Total RNA was isolated with the help of a QIAGEN kit (RNeasy Midi kit, cat#75144; RNeasy mini kit, cat#74104). First, a tissue stored in liquid nitrogen was ground with a cold mortar and pestle, and then lysed with 1 mL of a lysis buffer mixed with 10 µl of beta-mercaptoethanol. An equal volume of 70% ethanol was added to the lysis solution which was then loaded on the column of the kit to attach total RNA to the resin. The addition of 160 µl of RNase-free water separated and eluted total RNA. The absorbance of total RNA was read at 260 nm with a spectrophotometer to quantify the total RNA.

### Step 3) Reverse transcription

2 µg of the total RNA of each sample, prepared in Step 2, was mixed and reacted with a random primer at 70°C for 5 min, and then with a reverse transcription mix [2.5mM dNTP, 50U RNase Inhibitor, 200U M-MuLV reverse transcriptase, MBI fermentas, cat#EP0442), 1X M-MuLV reaction buffer] at 42°C for 2 hours to synthesize cDNA.

### Step 4) DD-PCR (Differential Display Polymerase Chain Reaction)

In order to isolate genes which were different in expression level between cancer cells and normal cells, each cDNA was amplified with a 200nM arbitrary primer (GenHunter Corporation, cat#H-AP) and a 200nM anchored primer (GenHunter Corporation, cat#H101-S) in the presence of 1U Taq polymerase (TaKaRa, cat#R001A). In this regard, [³⁵S]dATP (PerkinElmer, cat#NEG734H) was used for labeling. The PCR product thus obtained was mixed with 98% formamide dye and heated at 80°C for 5 min before being electrophoresed on 7M urea/6% polyacrylamide gel at 1,700 V for 3 hours. After being transferred onto 3MM Whatman paper, the gel was dried and exposed to an X-ray film. Development of the X-ray film was followed by reading.

### Step 5) Base sequence of DD-PCR product

As a result of DD-PCR, bands which were read to increase or decrease gene levels specifically in cancer tissues, compared to normal tissues, were selected. The bands were excised from the gel and the PCR products were purified with deionized water and precipitated in ethanol. The precipitated DNA was re-amplified with the same primers used in the DD-PCR. This amplified DNA was cloned in the cloning vector pGEM-T Easy vector system (Promega, cat#A1360) which was then transformed into E. coli (DH5α). Colonies of the transformed E. coli grown on LB-agar were separated and cultured again in an LB broth. Using a QIAGEN kit (QIAprep plasmid miniprep kit, cat#27104), plasmids were prepared from the E. coli. Treatment with the restriction enzyme EcoRI determined the sizes of the inserts. Using a big dye sequencing reaction mix (ABI), the inserts were base sequenced in an automated sequencer (ABI 3700). A homology search using BLAST (Basic Local Alignment Search Tool; Altschul et al. 1990; http://www.ncbi.nlm.nih.gov/BLAST/) program of the NCBI Gene Bank and the human EST Data Base was done for the base sequences to give gene information on each clone.

### EXAMPLE 2: Analysis of Gene Expression Using Microarray

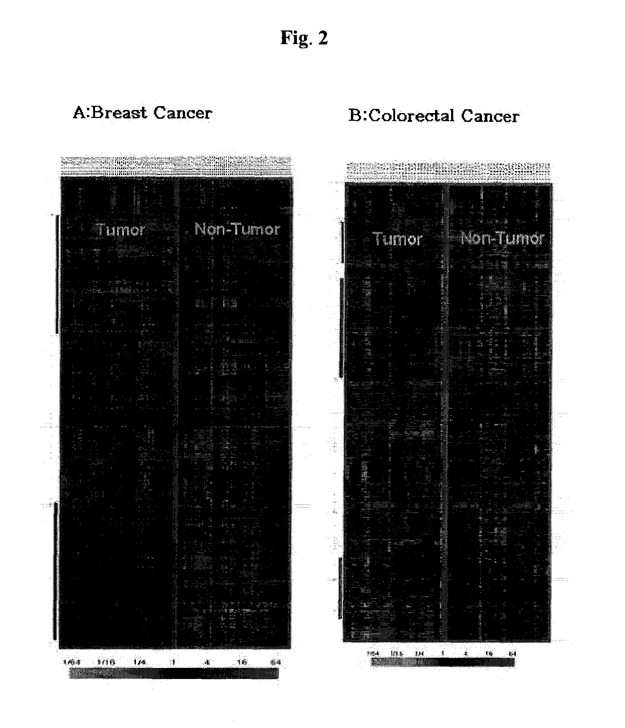

In order to select only genes the expression of which is highly specific for cancer tissues, out of about 3,000 genes cloned by DD-PCR, a large group of patients with cancer were secondarily screened. A human 3K cDNA chip on which the gene fragments cloned by DD RT-PCR were integrated was constructed to screen biopsy samples taken from 58 breast cancer patients and 32 colorectal cancer patients In the chip results obtained from 58 examples of patients with breast cancer and 32 examples of patients with colorectal cancer, genes the expression levels of which were increased or decreased twofold or higher in cancer tissues at significance compared to normal tissues were selected (FIG. 2).

### Step 1) Chip construction

A cDNA probe for use in chip construction was synthesized by PCR. A set of a forward primer 5'-CCG CGG GAA TTC GAT T-3'(SEQ ID NO.: 1) and a reverse primer 5'-GCC GCG AAT TCA CTA GTG-3' (SEQ ID NO.: 2) was used for the PCR. In order to amplify all of the genes equally, a PCR mix (0.3 pmole forward primer, 0.3 pmole reverse primer, 200 mM dNTP, 1 mM Tris-HCl, 5 mM KCl, 150 mM MgCl₂, 2.5unit Taq polymerase, ultra pure water) was aliquoted to each well of 96 well PCR plates, followed by adding 0.5 ml of each of the plasmid DNA templates carrying respective gene clones. In a DNA thermal cycler, PCR started with 94°C pre-denaturation for 5 min and continued for 40 cycles of denaturing temperature at 94°C for 30 sec, annealing temperature at 55°C for 30 sec and extending temperature at 72°C for 30 sec, finally followed by 72 °C extension for an additional 5 min. The PCR products thus obtained were cleaned up with a 384 well PCR purification filter manufactured by Millipore.

After being filtered, the cDNA was integrated on a GAPS II slide, manufactured by Corning, using a Microgrid II spotter, manufactured by BioRobotics (DNA chip construction was entrusted to Digital Genomic company).

The chip quality was determined through syto61 staining. The slide to be stained was treated with 0.1% SDS to remove excess probes. Treatment at 100°C for 2 min denatured probes such that syto61 dye was well intercalated thereinto. The slide was stained with syto61 dye for 5 min, followed by washing twice with distilled water to remove excess dye. The stained slide was read at 635 nm with an Axon laser scanner and visually analyzed to determine whether the chip was constructed correctly or not.

### Step 2) Tissue sampling

Colorectal cancer tissue samples were taken from 20 Korean adult male patients and 12 Korean adult female patients, who were 40 to 77 years old (LabGenomics). Breast cancer tissue sample were taken from 58 Korean adult female patients who were 28 to 71 years old (LabGenomics). A set of biopsies were sampled from tumorous tissue and normal tissue around the tumorous tissue in each patient.

### Step 3) Total RNA isolation

Tissue stored in liquid nitrogen (LN2) was ground with a cold mortar and pestle, and 100 mg of the tissue sample was mixed with 1 mL of TriZol reagent (Invitrogen, cat# 15596-018). The subsequent RNA isolation procedure was conducted according to the protocol provided by the manufacturer of the TriZol reagent. The RNA thus obtained was further purified using a QIAGEN kit (RNeasy Midi kit, cat#75144; RNeasy mini kit, cat#74104) according to the protocol provided by the manufacturer thereof. The RNA was quantified by measuring the absorbance at 260 nm using a spectrophotometer.

### Step 4) Sample preparation and labeling

DNA chip hybridization is a process in which cDNA synthesized from test RNA is reacted with the DNA integrated on the slide, that is, with the chip. For microarrays, 20∼50 µg of highly pure RNA was labeled with Cy3 or Cy5 fluorescent dye and subjected to reverse transcription. The labeling was conducted with Cy dye-coupled dUTP during PCR. For use as a control upon the hybridization between normal and tumor tissues, reference RNA was prepared. Out of various breast, colorectal and lung cancer cells, five cell lines were selected for each cancer in consideration of the characteristics of cancer tissues (breast cancer cell strains SK-BR-3, Hs578T, MCF-7, ZR-75-1 and MDA-MB-231, colorectal cancer cell strains COLO205 LoVo, WiDr, HT-29 and DLD-1, and lung cancer cell strains NCI-H23, NCI-H146, NCI-H69, NIH226 and A549). From each of the cell lines was isolated total RNA. Equal amounts of the total RNA were mixed to form a reference RNA as a control. This reference RNA was labeled with Cy3 while RNA obtained from breast cancer, colorectal cancer, and normal tissues were labeled with Cy5. Using a QIAquick PCR purification kit (QIAGEN, cat#28106), the salt, primers, detergents, proteins (enzymes) and RNA template used in PCR were removed from the cDNA obtained through reverse transcription. The purified, labeled cDNA was hybridized at 65°C for 16 hours with DNA on 3K chip. In this regard, unlabeled nucleotides, such as Cot-1 DNA and poly A RNA, were added as non-specific competitors so as to inhibit non-specific hybridization. The DNA chip was washed with a washing solution containing SSC to remove non-specific hybrids.

### Step 5) Gaining and qualification of chip image

Following chip hybridization, the chip was scanned with a GenePix 4000B scanner, manufactured by Axon, to obtain the fluorescence data from each spot. The fluorescence data was stored as TIFF files. Scanning results were analyzed in such a way that relative ratios of the numerical values read at the green and red wavelengths respectively obtained from Cy3 and Cy5 regions were used to determine the multiple by which the test group increased or decreased in gene expression compared to the control group.

### Step 6) Analysis of gene expression

In order to analyze or interpret DNA chip results, intensity values for each gene are needed. The GenePix program provided by Axon was used to convert the images obtained with the scanner into numerical values. After the intensity value of each spot was read, a statistic of numerical values for gene spots was obtained using error spots removed automatically or manually. Also, array quality control (QC) operations were conducted with regard to PMT, the laser power of Cy3 and Cy5, difference between signal and background, mean deviation of intensity values, intensity values between blocks, intensity values of all spots, and NF (no feature found) to examine whether the hybridization was well carried out on each experiment.

The total data was corrected using a Lowess normalization method lest a dye bias was formed between Cy5/Cy3 (Cleveland WS et al, 1992), 2,213 breast cancer genes and 1,940 colorectal cancer genes were analyzed, except for genes 20% or larger portions of which were missed.

Using the BRB array tools program (http://linus.nci.nih.gov/BRB-ArrayTools.html), clustering analysis between tissue samples was performed to prove the correctness of tissue sample origin and chip experiment. As a result, almost all tissue samples, with the exception of one breast cancer tissue sample and two colorectal tissue samples, were observed to be divided into two nodes: cancer tissue and normal tissue. These data indicate that the clinical samples used in this study were definitely divided into normal and cancer tissues and that the chip experiment was also correctly carried out.

In order to select the genes which show significant expression difference between breast or colorectal cancer tissues and normal tissues, SAM (significance analysis of microarrays; http://www-stat.stanford.edu/∼tibs/SAM/) was also performed. SAM is a useful technique by which only genes that show expression difference, attributed mainly to experimental results rather than to simple variation, at significance exceeding the error tolerance can be selected. At this time, the FDR (false discovery rate) was set to be zero on the basis of reliability of 99% or higher, so that the possibility of selecting genes based on statistical error was excluded.

### EXAMPLE 3: Assay for Cancer-Specific Gene Expression Using Real Time PCR

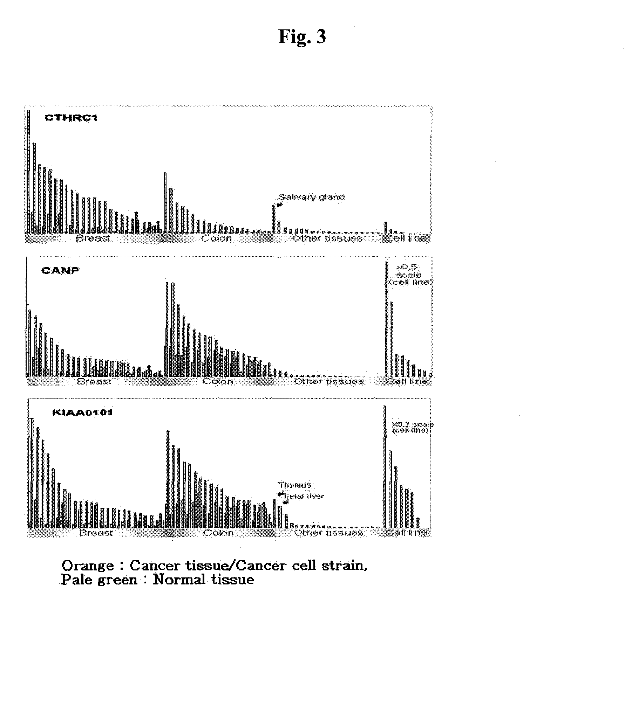

Of the genes discriminated using DNA chips, CANP, CTHRC1 and KIAA0101, supposed to be highly specific for cancer tissues, were selected. These candidate genes were measured for expression level in breast and colorectal cancer samples and various normal tissues and cancer cell lines to determine the breast or colorectal cancer-specific expression thereof (FIG. 3). In this regard, relative gene expression in breast cancer tissues, colorectal cancer tissues, 20 kinds of normal tissues (adrenal gland, brain, fetal brain, fetal liver, heart, kidney, liver, lung, placenta, prostate, salivary gland, skeletal muscle, spleen, testis, thymus, thyroid gland, trachea, uterus, small intestine, and spinal cord), a breast cancer cell line, a colorectal cancer cell line, and a lung cancer cell line was examined.

**[TABLE 1]**

| Class | Gene | Cancer | Average expression ratio of tumor vs. normal | Tumor over-expression fraction (>2 folds) |
|---|---|---|---|---|
| Hypothetic Oncogene | CTHRC1 | Breast | 10.9 | 88% |
| | | Colorectal | 22.0 | 90% |
| | CANP | Breast | 11.0 | 81% |
| | | Colorectal | 2.0 | 50% |
| | KIAA01 01 | Breast | 12.8 | 69% |
| | | Colorectal | 2.2 | 50% |

Data from the real time PCR indicate that CTHRC1 was 10-fold overexpressed in breast and colorectal cancer tissues on average and was expressed almost not at all in normal tissue. Over 80% of the tested patients with breast or colorectal cancer were found to overexpress CTHRC1 twofold in cancer tissues compared to normal tissues. CANP was overexpressed in the tissues and cell lines of breast and colorectal cancer, but was almost not found in normal tissues. Over 80% of the tested patients with breast cancer were observed to overexpress CANP twofold or higher in cancer tissues compared to normal tissues (11 folds on average). The patients with colorectal cancer who were observed to overexpress CANP twofold or higher amounted to 50% or higher. CANP was believed to be involved in cell proliferation, inferred from the fact that the expression level of the gene is high in cancer tissues and various cancer cell lines.

KIAA0101 was highly overexpressed in the tissues and cell lines of breast and colorectal cancer. Over 50% of the patients with breast or colorectal cancer were observed to overexpress KIAA0101 twofold or higher in cancer tissues compared to normal tissues. The expression level of this gene in the thymus, fetal liver, and small intestine of normal persons was almost equal to that in the normal tissues of patients with colorectal cancer. KIAA0101 is not expressed in other normal tissues. Generally, the expression behavior of KIAA0101 was found to be similar to that of CANP, which implies that KIAA0101 and CANP were functionally associated to each other.

### Step 1) Sample preparation

On the basis of the clinical information, obtained from DNA chip screening for the 56 pairs of breast cancer RNA samples and 32 pairs of colorectal cancer RNA samples, on cancer-generated sites, cancer progression, and biochemical report, and the expression results on DNA chips, 26 pairs of breast cancer samples and 20 pairs of colorectal cancer samples were selected. In order to quantify the expression level of the genes in normal organs other than cancer-generating organs, a tissue panel (Clontech Cat. # 636643) consisting of RNAs of 20 different normal tissues was used. The RNA obtained from the breast cancer cell lines Hs578T, SK-BR-3, and MCF7, the colorectal cancer cell lines COL0205, LOVO and WoDr, and the lung cancer cell lines A549, NCI-H146, and NIH226 was used for the measurement of gene expression in various cancer cell lines.

From the RNA obtained from clinical tissue samples, various cancer cell lines, and various normal tissues, cDNA was synthesized using Oligo(dT)20VN primer and SuperScript III (Invitrogen Cat. #18080-044). Reverse transcription was conducted with 1 mg of an RNA sample, Oligo(dT)20VN primer 2.5M, dNTP 0.5mM, MgCl₂ 5mM, 10mN DTT, 50U RNase Inhibitor, 200U SuperScript III, and 1X RT buffer (included in SuperScript III kit). Reaction at 50°C for one hour resulted in the synthesis of cDNA. Heating at 85°C for 5 min terminated the synthesis. The cDNA thus obtained was aliquoted in small amounts and stored at -70°C.

### Step 2) Primer preparation

On the basis of the cDNA base sequences of CANP, CTHRC1 and KIAA0101, sets of primers respectively specific for CTHRC1, CANP and KIAA0101 were synthesized. A set of primers for a beta-actin gene (ACTB) for use as an endogenous control was synthesized on the basis of the mRNA base sequence (NM_001101) searched with the NCBI GeneBank database. Using the Primer Express program provided by Applied Biosystems, all sets of the primers were designed to meet general PCR primer requirements, to have a Tm value of about 80°C and to allow the PCR products to have a length of 100 bp. Primers specifically used for the genes are as follows.
ACTB-for: 5'-GGCATTGCCGACAGGATG-3' (SEQ ID NO.: 3)
ACTB-rev: 5'-CTCAGGAGGAGCAATGATCTTGAT-3'(SEQ ID NO.: 4)
CTHRCl-for: 5'-TTGGGAAAATTGCGGAGTGT-3' (SEQ ID NO.: 5)
CTHRCl-rev: 5'-GCCGAAGTGAGCCACTGAAC-3' (SEQ ID NO.: 6)
CANP-for: 5'-TCGGTCTGACATAGGTGAATTTGA-3'(SEQ ID NO.: 7)
CANP-rev: 5'-TCATCCACCATGGACTGTTTTC-3' (SEQ ID NO.: 8)
KIAA0101-for:5'-TCATCGAGGAAAGCTGAAAATA-3'(SEQ ID NO.:9)
KIAA0101-rev:5'-AATTCCTTTTTGCCACTTGG-3'(SEQ ID NO.: 10)

### Step 3) Reaction condition

SYBR Green assay was conducted with 25 µl of a solution containing 12.5 µl of an SYBR Green PCR master mix (Applied Biosystems Cat. # 4309155), cDNA, a forward primer, and a reverse primer.

cDNA was added in an amount such that the total RNA used for reverse transcription amounted to 50 ng. As for the primers, the forward primer and the reverse primer were used in amounts selected from among 3x3 concentration combinations of 100nM, 300nM, and 900nM that showed the best gene amplification without non-specific synthesis. Each of the primers specific for beta-actin, CTHRC1, CANP, and KIAA0101 were used in a concentration of 300 nM. Negative control samples lacking cDNA were also prepared for all sets of the primers to examine the production of non-specific products. The same experiments were repeated two or three times to reduce the experimental error attributable to pipetting error and variation between wells. PCR started with 50°C annealing for 1 min and 95°C denaturing for 10 min and was carried out with 40-50 cycles of denaturing temperature at 95°C for 15 sec and annealing temperature at 60°C for 1 min. After the completion of PCR, a dissociation experiment was performed according to the protocol provided by the apparatus, so as to examine non-specific amplification.

### Step 4) Measurement of relative gene expression level

The concentration of the cDNA was determined within a range that did not deviate from the Ct (cycle of threshold) value of the clinical sample to be tested from the standard curve. As a sample for a standard curve, the cDNA from a mixture of the RNAs prepared form tissue samples was used. Experiments for standard curves were repeated three times within the same plate for each reaction. If it was remarkably different from the other, the Ct value of one reaction was excluded. Using the average Ct value of the remaining 2 or 3 reactions, a standard curve was plotted. In this regard, the threshold value was controlled such that the standard curve had a correlation coefficient (R2) of near 1 and a slope of near 3.3.

Since the accurate amount of a specific gene within the cDNA for the standard curve was not known, the gene amount determined by this assay might be an expression level relative to the standard sample. Ct values for each sample were determined using the threshold value set when the standard curve was drawn. By comparison between the Ct value and the standard curve, the gene level was obtained. cDNA concentration variations according to sample were normalized to determine the relative gene expression level of each sample.

The difference in cDNA concentration among samples is attributed largely to the difference in reverse transcription efficiency. That is, although the reverse transcription is carried out under the same condition, the amount of the cDNA synthesized differs from one well to another due to the variation between wells of the PCR apparatus. Because the step of isolating only cDNA or measuring the concentration of the cDNA after reverse transcription lacks, the cDNA concentration difference between samples attributable to reverse transcription efficiency can be normalized with an endogenous control. The house-keeping gene beta-actin was used as the endogenous control. The relative concentration of the endogenous control in each cDNA was determined using the above-mentioned method, followed by calculating the normalization fold for each sample. On the assumption that the endogenous control is expressed in the same level in all samples, the relative amount of each cDNA is proportional to the expression level of the endogenous control. Therefore, the normalization fold for correcting the cDNA difference is the reciprocal of the relative amount of the endogenous control.

When the concentration difference of DNA is corrected, the gene amount obtained using the given standard curve is multiplied by the normalization fold to obtain relative gene expression levels.

### EXAMPIE 4: Analysis of Effect of Overexpresion on Cell

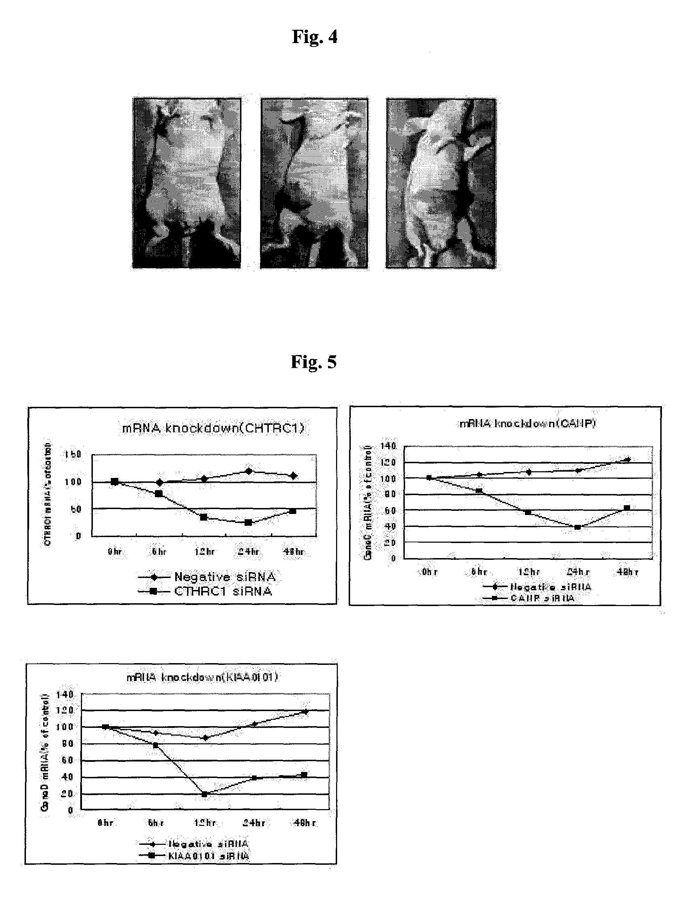

In order to examine the effect of the overexpression of the cancer-specific CTHRC1, KIAA0101 and CANP on oncogenesis, cell strains which overexpressed corresponding genes were prepared. The injection of the transformed cell strains caused the nude mice to form distinct tumors 1.5-2 cm long at the sites of the injection (FIG. 4). These data indicate that when overexpressed, the respective genes play an important role in oncogenesis.

### Step 1) Cloning of full-length gene

### 1-1. cloning of full-length CTHRC1

CTHRC1 consists of a single ORF (open reading frame) 732bp long. For use in the amplification of CTHRC1, a set of primers specific for CTHRC1 were designed on the basis of the nucleotide sequence NM_138455(NCBI) such that the PCR comprised the overall ORF.
CTHRCl-For 3'-CTGACCACGTTCCTCTCCTCGGTCT-5' (SEQ ID NO.: 11)
CTHRC1-Rev 3'-TGTCATTTAAGTGAACCATTCCAAGGCA-5' (SEQ ID NO.: 12)

PCR was carried out while a cDNA library synthesized from the total RNA of breast cancer tissues served as a template. Each of the 3' and 5' primers was used in an amount of 10 pmole per 100 ng of the template cDNA. PCR was performed with 35 cycles of denaturing temperature at 94°C for 30 sec, annealing temperature at 68°C for 2 min and extending temperature at 72°C for 7 min. The PCR product DNA was electrophresed on 1.5% agarose gel to determine the size thereof. Treatment with restriction enzymes was useful to examine the correct amplification of a gene of interest. The amplified, full-length cDNA was cloned in pGEM T easy vector (Promega, Cat#A1360) to secure the full-length gene.

### 1-2. Cloning of full-length CANP

CANP consists of a single ORF 2205 bp long. For use in the amplification of CANP, a set of primers specific for CANP were designed on the basis of the nucleotide sequence NM_198947(NCBI) such that the PCR comprised the overall ORF.
CANP-For 5'-CCTTGTTTCTCCATCTTATCGAGT-3' (SEQ ID NO.: 13)
CANP-Rev 5'-ATTTTCTCCCATTCCGTAGGTTGT-3' (SEQ ID NO.: 14)

PCR was carried out while a cDNA library synthesized from the total RNA of breast cancer tissues served as a template. Each of the primers was used in an amount of 10 pmole per 100 ng of the template cDNA. PCR was performed with 35 cycles of denaturing temperature at 94°C for 30 sec, annealing temperature at 68°C for 2 min and extending temperature at 72°C for 7 min. The PCR product DNA was electrophresed on 1.5% agarose gel to determine the size thereof. Treatment with restriction enzymes was useful to examine the correct amplification of a gene of interest. The amplified, full-length cDNA was cloned in pGEM T easy vector (Promega, Cat#A1360) to secure the full-length gene.

### 1-3. Cloning of KIAA0101

KIAA0101 consists of a single ORF 336 bp long. For use in the amplification of KIAA0101, a set of primers specific for KIAA0101 were designed on the basis of the nucleotide sequence NM_014736(NCBI) such that the PCR comprised the overall ORF.
KIAA0101 For 5'-CTCGGCTGGGAAGTCAGTTCGTTCTC-3' (SEQ ID NO.: 15)
KIAA0101 Rev 5'-AAGCCACTGTGCCCACCATGATTCTAT-3' (SEQ ID NO.: 16)

PCR was carried out while a cDNA library synthesized from the total RNA of breast cancer tissues served as a template. Each of the 3' and 5' primers was used in an amount of 10 pmole per 100 ng of the template cDNA. PCR was performed with 35 cycles of denaturing temperature at 94°C for 30 sec, annealing temperature at 68°C for 2 min and extending temperature at 72°C for 7 min. The PCR product DNA was electrophresed on 1.5% agarose gel to determine the size thereof. Treatment with restriction enzymes was useful to examine the correct amplification of a gene of interest. The amplified, full-length cDNA was cloned in pGEM T easy vector (Promega, Cat#A1360) to secure the full-length gene.

### Step 2) Construction of expression vector

In order to clone the cDNAs of CTHRC1, CANP and KIAA0101, the restriction enzyme sites of which were identified, in a pDOR221 vector (Invitrogen, Cat# 12535-019), a 29 bp long *att*B site was attached to both ends of the PCR product. According to the Gateway Technology manual (Invitrogen, 22 September 2003, 25-0522), PCR was carried out twice to attach an attB adapter to both ends of the PCR product. The *att*B-PCR product was purified with a QIAGEN PCR purification kit (QIAGEN, Cat# 28106), and then cloned into a *att*P-containing donor vector (pDONR221) using BP Clonase Enzyme(Invitrogen, Cat# 11789-013). Base sequencing analysis was done on the overall genes so as to examine whether the cording region of each gene was correctly cloned. The genes cloned into pDONR221 vectors were transferred into the pcDNA-DEST40 (Invitrogen, Cat# 12274-015) Gateway vector for transformation, with the aid of LR Clonase(Invitrogen, Cat# 11791-019).

### Step 3) Transformation of cells

The plasmids pcDNA-DEST40/CANP, pcDNA-DEST40/KIAA0101, and pcDNA-DEST40/CTHRC1, each carrying a gene-specific gene, were transfected into a 293 human embryonic kidney cell line (ATCC, cat#CRL1573) using LipofectamineTM 2000 (Invitrogen, cat#11668), and then treated with Geneticin (Invitrogen, cat#11811-023) for about three weeks to select the cells into which the plasmid was introduced. Single colonies grown from single cells were taken and cultured in the following 96 well plates.

To identify the protein expression of the newly introduced expression vector, proteins were extracted from the transformed cell lines and subjected to Western blotting analysis. Because being conjugated to each of the target proteins, a V5 epitope and a 6x His tag were measured for expression level using an Anti-V5-HRP antibody (Invitrogen, cat# R961-25) and an Anti-His(C-term)-HRP antibody (Invitrogen, cat# R931-25) respectively, so as to indirectly identify the intracellular expression of the target protein.

In order to secure cell lines which were uniformly kept at expression rate, the selected overexpression cells were diluted and aliquoted to a 96 well plate in such a way that only one cell was assigned to one well. Following incubation for 2-3 days, each well was measured for the number of clones under a microscope. The wells which had formed only one clone per well were marked and incubated for an additional one week. After being detached through treatment with trypsin, clones were detached from the marked wells and transferred into 48 well, 24 well, and 6 well plates to obtain a large number of cells.

### Step 4) Oncogenicity in animal

The overexpression cells obtained through step 3 were subcutaneously injected into 10 nude mice which were all five weeks old. 20 days after the injection, all of the 10 mice were observed to form distinct tumors 1.5-2 cm in size at the injection sites.

### EXAMPLE 5: Analysis for Function of Cancer-Specific Genes Using RNAi (RNA interference)

The effect of the genes expressed specifically in cancer tissues on the function of cancer cells was examined. In this regard, while the genes which had been identified to show cancer-specific expression were specifically inhibited from expression by use of RNA interference (RNAi), an examination was made of how the cells were affected through real time PCR.

### 1. Specific suppression of gene expression using RNAi

The introduction of siRNA was observed to lead to the specific knockdown of corresponding genes through real time PCR (FIG. 5). A test group into which siRNA specific for CTHRC1, CANP or KIAA0101 was introduced, a control into which scrambled negative siRNA non-specific for the genes were introduced, and a control treated with no siRNA were compared. Only the test group which had the gene-specific siRNA introduced thereinto showed the specific suppression of the corresponding genes. 6 hours after treatment with CTHRC1 siRNA, the test group showed 30% higher knockdown of CTHRC1 mRNA expression as compared to the control. 24 hours after the treatment, the test group showed 80% higher knockdown of the corresponding mRNA expression as compared to the control. As for CANP siRNA, it induced 70% higher knockdown of CANP mRNA expression 6 hours after the treatment therewith, as compared to the control. KIAA0101 siRNA showed 80% knockdown of KIAA0101 expression from 12 hours after the treatment therewith. The knockdown effect of siRNA-specific gene expression was maintained for 48 hours or longer. There was no significant difference in the mRNA level of the targeted genes between the control groups treated with and without negative siRNA.

### 2. Effect of suppression of gene expression on cell death induction and cell proliferation reduction

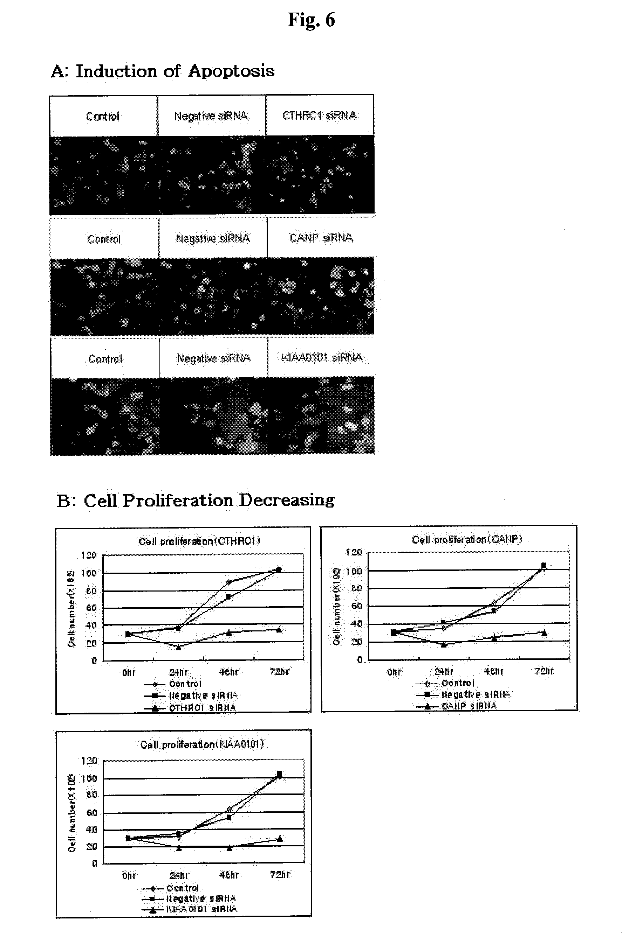

In order to examine the toxicity of transfection and the effect of gene knockdown, cells were observed for viability using EthD-1 fluorescent dye, which is selective for dead cells (FIG. 6A). Also, viable cells were counted according to time to monitor the effect of gene suppression on cell proliferation (FIG. 6B).

When the count of dead cells was examined with fluorescent images obtained by EthD-1 fluorescent dye, which is selective for dead cells, the suppression of CTHRC1 gene caused 50% higher counts of cells to undergo cell death as compared to the control. Whereas the untreated control threefold increased in cell count for 72 hours, the test group in which the expression of the CTHRC1 gene was suppressed did proliferate almost not at all for 72 hours. These results indicate that the suppression of CTHRC1 leads to the induction of cell death and the suppression of cell proliferation.

When the expression of the CANP gene was suppressed, the count of the cells undergoing cell death increased 6 hours after treatment with siRNA and was found to be 40% higher 24 hours after the treatment, as compared to the control. Whereas the untreated control threefold increased in cell count for 72 hours, the test group in which the expression of the CANP gene was suppressed did proliferate almost not at all for 72 hours. These results indicate that the suppression of CANP leads to the induction of cell death and the suppression of cell proliferation.

In the case of KIAA0101 siRNA, although a significant change in mRNA level was detected 12 hours after treatment with KIAA0101 siRNA, cell death was observed to start from 6 hours after the treatment as analyzed by EthD-1 staining. Upon the suppression of KIAA0101 gene, over 30% of the cell counts underwent cell death and no cell proliferation was observed until 72 hours. These results indicate that the suppression of KIAA0101 leads to the induction of cell death and the suppression of cell proliferation.

As for the control group which was treated with negative siRNA, it did not undergo cell death according to the introduction of siRNA, but showed cell proliferation to the same extent as in the untreated control group. Taken together, the data obtained in above experiments demonstrate that the induction of cell death and the suppression of cell proliferation according to the introduction of CTHRC1, CANP or KIAA0101 siRNA are attributed specifically to the suppression of each gene.

### 3. Induction of apoptosis by gene suppression

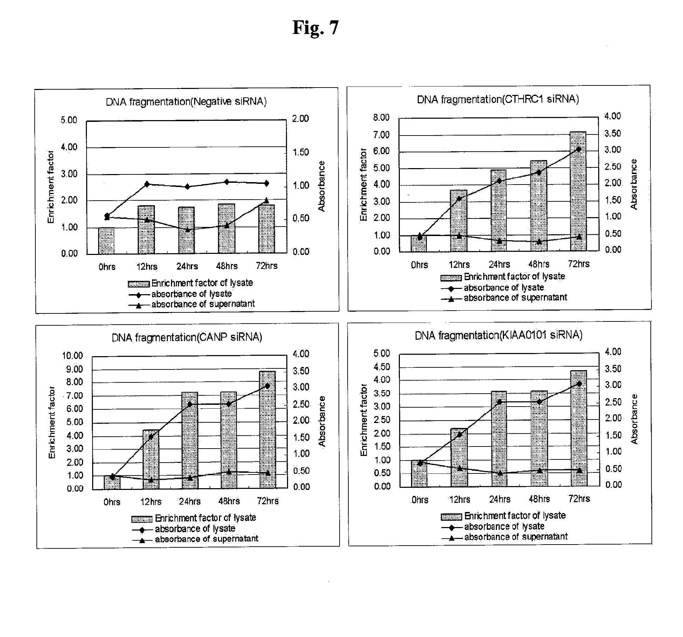

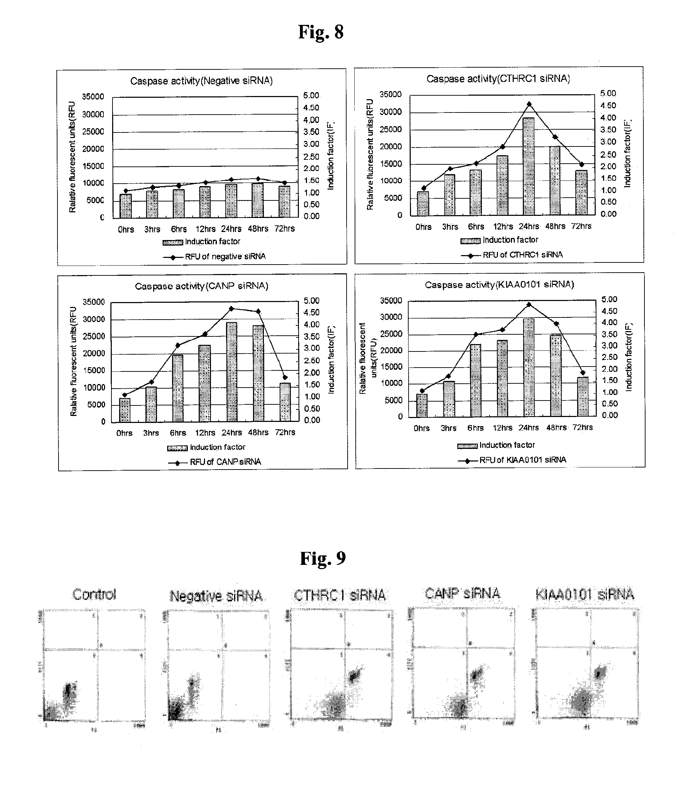

To determine the type of the cell death induced by gene suppression, the activity of caspase, an apotosis-related enzyme (FIG. 8), DNA fragmentation (FIG. 7) and membrane alteration were analyzed, and the degrees to which apoptosis and necrosis were generated were monitored according to time (FIG. 9).

When the CTHRC1 gene was suppressed, the caspase activity started to increase just after the introduction of siRNA and was found to fourfold increase, as compared to the control, 24 hours after the introduction at which the cell death was maximized. DNA fragmentation also increased gradually and was found to fivefold increase, as compared to the control, 72 hours after the introduction. As apoptosis proceeds, the PS (phosphatidyl serine) of cell membranes was exposed to the outside.

When the CANP gene was suppressed, the caspase activity started to increase 6 hours after the introduction of siRNA and was found to fourfold increase 24 hours after the introduction, as compared to the control. DNA fragmentation also eightfold increased 72 hours after the introduction, as compared to the control. As apoptosis proceeds, the PS (phosphatidyl serine) of cell membranes was exposed to the outside.

When the KIAA0101 gene was suppressed, the caspase activity started to increase just after the introduction of siRNA and was found to 45-fold increase24 hours after the introduction, as compared to the control. DNA fragmentation was also found to threefold increase72 hours after the introduction, as compared to the control. As apoptosis proceeds, the PS (phosphatidyl serine) of cell membranes was exposed to the outside.

The degree of the apoptosis, which was identified by the features of caspase activity, DNA fragmentation and membrane alteration, was observed to increase in the order of KIAA0101, CANP, and CTHRC1. The group treated with negative siRNA did not undergo the siRNA introduction-attributable cell death almost at all, implying that the induction of cell apoptosis according to the introduction of CTHRC1, CANP or KIAA0101 siRNA results specifically from the suppression of each gene. Also, these results show that the suppression of the expression of the novel cancer-specific genes CTHRC1, CANP and KIAA0101 causes cell death, probably apoptosis, indicating that the novel cancer-specific genes CTHRC1, CANP and KIAA0101 play an important role in cell viability.

### 4. Effect of gene suppression on cell division

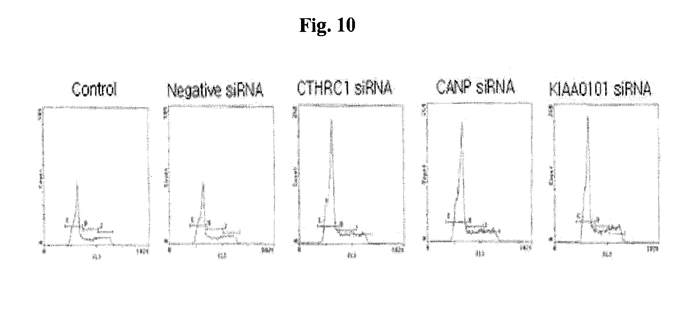

In order to examine the cell proliferation-suppressive effect induced by gene suppression, a cell cycle change was measured with PI (propidium iodide) (FIG. 10).

The cells treated with CTHRC1 siRNA were about 20% decreased in the proportion of cells in S phase and G2/M phase and about 20% increased in the proportion of cells in G0/G1 phase 3 hours after the treatment, as compared to the control. The cells treated with CANP siRNA, also, was 120% decreased in the proportion of cells in S phase to the maximum and 60% decreased in the proportion of cells in G2/M phase whereas they were 5 to 30% increased in the proportion of cells in G0/G1 phase, as compared to the control. The cells treated with KIAA0101 siRNA were found to 125% decrease in the proportion of cells in S phase to the maximum and 50% in the proportion of cells in G2/M and 10-40% increase in the proportion of cells in G0G1 phase, as compared to control.

It is believed that when CTHRC1, CANP and/or KIAA0101 are expressed in a low level, apoptosis is induced to arrest the cells in G1 phase, thereby suppressing the cell proliferation. Cells treated with negative siRNA showed neither the suppression of cell proliferation nor cell death according to the introduction of siRNA. Consequently, the change in cell cycle according to the introduction of CTHRC1, CANP or KIAA0101 siRNA results specifically from the suppression of each gene.

### Step 1) siRNA (small interfering RNA) construction

The overall mRNA sequences of CTHRC1, CANP, and KIAA0101 were obtained using the NCBI GeneBank program (http://www.ncbi.nlm.nih.gov/entrez/). siRNA was designed with the aid of the Block-IT^{™} RNAi designer of Invitrogen (http://www.invitrogen.com/) and synthesized by Invitrogen. siRNA base sequences for each gene are as follows.

**[TABLE 2]**

| Gene | Accession number | siRNA sequence (5' to 3') forward/reverse |
|---|---|---|
| CTHRC1 | NM138455 | UUAUAGCUUCAAUGGGAAGAGGUCC (SEQ ID NO.: 17)/ GGACCUCUUCCCAUUGAAGCUAUAA(SEQ ID NO.: 18) |
| CANP | NM198947 | CCAGAGUCUGAUACAGUCUAAGAAA (SEQ ID NO.: 19)/ UUUCUUAGACUGUAUCAGACUCUGG(SEQ ID NO.: 20) |
| KIAA0101 | NM014736 | AUGAAACUGAUGUCGAAUUAGUGGC (SEQ ID NO.: 21)/ GCCACUAAUUCGACAUCAGUUUCAU(SEQ ID NO.: 22) |

### Step 2) Transfection of siRNA

Experiments were conducted with the breast cancer cell lines MCF-7 and SK-BR3, the colorectal cancer cell lines WiDr and HCT116, and the lung cancer cell line A549. Each of the cell lines was aliquoted into 48-well plates in an amount of 3×10⁴ cells/well. 16 hours later, siRNA for each gene or negative control siRNA (Invitrogen, cat#12935-100) was, along with fluorescent oligo (Invitrogen, cat#13750-062), mixed with Lipofectamine^{™} 2000 (Invitrogen, cat#11668-027) and added to the culture medium in which the cell lines had been grown. At intervals of 24, 48, and 72 hours, the cells lines were monitored according to the concentration of siRNA. For use, siRNA and fluorescent oligo, stored in 20 nM stock solutions, were diluted in pH 7.4 buffer containing 100mM KOAc, 30mM HEPES-KOH, and 2mM MgOAc.

### Step 3) Change in mRNA level by gene knockdown

To measure mRNA knockdown results through an RNAi technique, cell lines were aliquoted into 12-well plates in an amount of 2×10⁵ cell/well and cultured for 16 hours. Subsequently, 10, 20, 50, and 100nM siRNA were mixed with 1 ml of lipofectamine and added to the culture media in which the cells had been grown, followed by extracting total RNA 6, 12, 24, and 48 hours after the addition. RNA was extracted with an RNeasymicro kit (QIAGEN, cat#74004). Treatment with DNase (QIAGEN, cat#79254) removed a trace amount of genomic DNA from the RNA sample.

Using quantitative real time PCR, the gene knockdown effect attributed to RNAi was measured. The extracted RNA was subjected to reverse transcription using SuperScript III reverse transcriptase (Invitrogen, cat#18080-044) and poly dT primer. The PCR product was detected by SYBR green assay. For this assay, 12.5 µl of SYBR green PCR master mix (Applied Biosystems, cat#4309155) was mixed with cDNA, a forward primer, and a reverse primer to a final volume of 25 µl. cDNA was added in an amount such that the total RNA used for reverse transcription amounted to 50 ng. Each of the primers was added in an amount of 300 ng. The cDNA concentration difference between samples attributable to reverse transcription efficiency was normalized with the endogenous control beta-actin.

### Step 4) Change in phenotype by gene knockdown

In order to examine the toxicity of transfection and the effect of gene knockdown, cells were observed for viability using EthD-1 fluorescent dye (Ethidium homodimer-1, Sigma, cat#E1169), which is selective for dead cells. Cells were treated with 2 mM EthD-1. The trasnfection result of siRNA was observed with a fluorescent microscope (Olympus IX51). Fluorescent images were obtained from fluorescent oligo at FITC wavelengths (ex=494nm, em=519nm ) and from EthD-1 at Texas-Red wavelengths (ex=528nm, em=617nm).

### Step 5) Change in cell proliferation by gene knockdown

Cell lines were aliquoted into 96-well plates in an amount of 3×10³ cell/well and cultured for 16 hours. Subsequently, 10, 20, 50, and 100nM siRNA for each gene or the negative control siRNA was mixed with 0.1 ml of Lifofectamine and added to culture media in which the cells had been grown. 24, 48, and 72 after the addition, cell counting assay was performed. siRNA-transfected cells were detached with 50 µl of trypsin (GIBCO, cat#15090-046) and damaged cells were stained with 50 µl of trypan blue stain (GIBCO, cat#15250-061) to count viable cells with a hemocytometer.

### Step 6) Measurement of apoptosis

### 6-1. measurement of DNA fragmentation

A cell death detection ELISA kit (Roche, cat#1774425) was used to examine the DNA fragmentation pattern of the cells which underwent apoptosis or necrosis. After siRNA was introduced into the cell lines to conduct RNAi, the supernatant in which cells in necrotic cell death mode were contained was separated and the cells in apoptotic cell death mode were lysed to isolate the DNA thereof. The DNA was placed into a straptavidin-coated microplate and reacted with an anti-histon biotin antibody and an anti-DNA POD antibody. After color development with the peroxidase substrate ABTS, absorbance at 405 nm was measured. The absorbance values left after a background value was deduced from the measured absorbance values were used to calculate enrichment factors (ratio of mono/oligonucleosome released into cytoplasm). From these parameters, the cell death attributable to gene knockdown was quantitatively analyzed.

### 6-2. Measurement of caspase activity

A homogeneous caspase assay kit (Roche, cat#3005372) was used to measure the caspase activity of the cells in which apoptosis was induced. siRNA was introduced into cell lines to perform RNAi and reacted with the caspase substrate DEVD-R110 coupled with the fluorescent material Rhodamine 110. Fluorescence was measured with a fluorimetric reader equipped with a 470-500nm (excitation wavelength) filter and a 500-560nm (emission wavelength) filter. Result analysis was conducted in such a way that absorbance values (relative fluorescence units, RFU) left after a blanc value was deduced from measured absorbance values were used to calculate induction factors (IF, ratio of caspase activity of test group vs. control). From these parameters, caspase activity changes attributable to gene knockdown were quantitatively analyzed.

### 6-3. Measurement of membrane alteration

In cells which undergo apoptosis, PS (phosphatidyl serine) is exposed to the outside of the cell membrane. The exposed PS was detected with Annexin-V, which specifically bind to PS, using an Annexin V-FITC apoptosis kit (BD, cat#K2025-1). Cell death was induced in cell lines through introduction of siRNA thereinto. The cells were reacted with FITC-coupled Annexin-V and counted using a flow cytometer manufactured by BectonCoulter. At this time, PI (propidium iodide), which is specific for DNA, was also incubated along with the Annexin-V, so as to distinguish apoptotic cells in later stage from necrotic cells.

### Step 7) Measurement of cell cycle change

siRNA was transfected into cell lines to knockdown corresponding genes, followed by fixation with 70% ethanol. The DNA was stained with DNA-specific PI (propidium iodide) and measured using a flow cytometer. Phases of cell cycle were distinguished according to DNA level.

### Industrial Applicability

As described hitherto, breast or colorectal cancer can be significantly diagnosed by detecting the expression of a gene selected from a group consisting of CTHRC1, CANP, KIAA0101 and combinations thereof and can be effectively prevented or treated by inhibiting the expression and activity of the gene.

### CLAUSES

Clause 1: A diagnostic marker for breast or colorectal cancer, comprising a gene selected from a group consisting of CTHRC1 (Collagen Triple Helix Repeat Containing 1), CANP (Cancer-Associated Nucleoprotein), KIAA0101 and combinations thereof.

Clause 2:A diagnostic maker for breast or colorectal cancer, comprising a protein selected from a group consisting of CTHRC1, CANP, KIAA0101 and combinations thereof.

Clause 3: A kit for detecting a diagnostic marker for breast or colorectal cancer, comprising an agent capable of measuring the mRNA level of a gene selected from a group consisting of CTHRC1, CANP, KIAA0101 and combinations thereof.

Clause 4: A kit for detecting a diagnostic marker for breast or colorectal cancer, comprising an agent capable of measuring the protein level of a gene selected from a group consisting of CTHRC1, CANP, KIAA0101 and combinations thereof.

Clause 5: A method for detecting a diagnostic marker for' breast or colorectal cancer, comprising comparing expression levels of a gene selected from a group consisting of CTHRC1, CANP, KIAA0101 and combinations thereof between a test bio-specimen and a control specimen.

Clause 6: The method according to clause 5, wherein the bio-specimen is selected from a group consisting of tissues, cells, whole blood, sera, plasma, saliva, phlegm and urine.

Clause 7: A pharmaceutical composition for the prophylaxis and treatment of breast or colorectal cancer, comprising an siRNA (small interfering RNA) sequence, capable of inducing RNA interference specific for a predetermined gene, composed of an antisense RNA strand complimentary to the mRNA of the predetermined gene and a corresponding sense RNA strand, said predetermined gene being selected from a group consisting of CTHRC1, CANP, KIAA0101 and combinations thereof.

Clause 8: The pharmaceutical composition according to clause 7, wherein the siRNA is composed of a set of nucleotides represented by SEQ ID NOS. : 17 and 18, SEQ ID NOS. : 19 and 20, or SEQ ID NOS.: 21 and 22.

Clause 9: A pharmaceutical composition for the prophylaxis or treatment of breast or colorectal cancer, comprising an antibody specific for a protein selected from a group consisting of CTHRC1, CANP, KIAA0101 and combinations thereof.

Clause 10: A method for suppressing the cell proliferation of breast or colorectal cancer or inducing apoptosis in breast or colorectal cancer cells, comprising reducing the intracellular mRNA or protein level or protein activity of a gene selected from a group consisting of CTHRC1, CANP, KIAA0101 and combinations thereof.

Clause 11: A method for suppressing the cell proliferation of breast or colorectal cancer or inducing apoptosis in breast or colorectal cancer cells, comprising Administering the composition of one of clause 7 to 9.

## Claims

1. A CANP gene, for use as a diagnostic marker for breast cancer or colorectal cancer.

2. A protein encoded by the CANP gene, for use as a diagnostic marker for breast cancer or colorectal cancer.

3. A pair of primers or a probe, for use as a diagnostic marker for breast cancer or colorectal cancer, wherein the primers or probe are used to measure the mRNA level of the CANP gene, preferably wherein the primers or the probe are specific for a region of the CANP gene.

4. An antibody, for use as a diagnostic marker for breast cancer or colorectal cancer, wherein the antibody is specific for the protein encoded by the CANP gene and is used to measure the level of protein encoded by the CANP gene.

5. A method for detecting a diagnostic marker for breast cancer or colorectal cancer, comprising comparing expression levels of a CANP gene in a test bio-specimen with a control specimen.

6. The method according to Claim 5, wherein the bio-specimen is selected from the group consisting of tissues, cells, whole blood, sera, plasma, saliva, phlegm and urine.

7. A pharmaceutical composition comprising a siRNA (small interfering RNA) sequence, for use in prophylaxis or treatment of breast cancer or colorectal cancer, wherein the siRNA is capable of inducing RNA interference specific for a CANP gene, and is composed of an antisense RNA strand complimentary to the mRNA of the CANP gene and a corresponding sense RNA strand.

8. A pharmaceutical composition for use according to Claim 7, wherein the siRNA is composed of a set of nucleotides represented by SEQ ID NOS.: 17 and 18, SEQ ID NOS.: 19 and 20, or SEQ ID NOS.: 21 and 22.

9. A pharmaceutical composition comprising an antibody specific for the protein encoded by the CANP gene, for use in prophylaxis or treatment of breast cancer or colorectal cancer.

10. The CANP gene for use according to Claim 1, or the protein encoded by the CANP gene for use according to Claim 2, or the pair of primers or the probe for use according to Claim 3, or the antibody for use according to Claim 4, or the method according to Claims 5 or 6, or the pharmaceutical composition for use according to any of Claims 7-9, comprising the genes:
CANP and CTHRC1;
CANP and KIAA0101; or
CANP, CTHRC1 and KIAA0101.
